Europäisches Patentamt

European Patent Office

Office européen des brevets

(11) Publication number: **0 340 934**
**A1**

(12) **EUROPEAN PATENT APPLICATION**

(21) Application number: **89303741.6**

(51) Int. Cl.⁴: **C12N 5/00**

(22) Date of filing: **14.04.89**

(30) Priority: **14.04.88 IE 1133/88**
**14.04.88 IE 1134/88**
**14.04.88 IE 1135/88**

(43) Date of publication of application:
**08.11.89 Bulletin 89/45**

(84) Designated Contracting States:
**AT BE CH DE ES FR GB GR IT LI LU NL SE**

(71) Applicant: **UNIVERSITY COLLEGE DUBLIN**
**Belfield**
**Dublin 4(IE)**

(72) Inventor: **Gordon, Ian**
**1 College Lands**
**Saggart County Dublin(IE)**
Inventor: **Lu, Ke Huan**
**94 Cherrymount**
**Clonmel County Tipperary(IE)**

(74) Representative: **Goldin, Douglas Michael et al**
**J.A. KEMP & CO. 14, South Square Gray's Inn**
**London WC1R 5EU(GB)**

(54) In vitro culture of bovine embryos.

(57) A method for the in vitro culture of the early bovine zygote to the late morula or blastocyst stage comprises incubating intact Cumulus-Oocyte-Complex under non-static conditions in a medium containing greater than 10% by weight of heat-inactivated oestrous cow serum and greater than $4 \times 10^6$ additional follicle cells until the oocytes reach maturation, fertilising the oocytes with capacitated bull sperm and co-culturing the zygotes thereby obtained on a monolayer of cumulus cells for 6-9 days. The zygotes are then evaluated and are either used for fresh transfers to a recipient cow or heifer or, alternatively, are stored frozen in liquid nitrogen for subsequent use.

EP 0 340 934 A1

## IN VITRO CULTURE OF BOVINE EMBRYOS

This invention relates to in-vitro fertilisation (IVF) and embryo transfer in cattle. More particularly, the invention relates to a method of in-vitro culture of the early bovine zygote (2-4 cell stage).

Successful in vitro fertilisation in mammals was first achieved in 1954. However, relatively reliable and reproducible methods of IVF have only been developed in the last decade and currently only apply to a small number of species.

The basic procedures employed in embryo transfer in cattle are now well-developed with considerable numbers of pregnancies being established by this technique each year.

Until recently calves born after IVF have been the result of in-vivo matured oocytes transferred surgically to the oviducts of recipient cattle or after incubation in rabbit oviducts. Critser, E.S. et al. (-(1986) Biol. Reprod., 34 (Suppl. 1) 286 (Abs)) reported a pregnancy originating from in-vitro matured oocytes fertilised in-vitro, then cultured in the ligated sheep oviduct before being transferred to a recipient heifer.

Many different media have been employed in IVF studies. Fertilisation in mammals takes place in a closely controlled environment within the ampulla of the fallopian tube. In setting up a suitable IVF technique, the culture system must be capable of providing the matured oocyte and the capacitated sperm with an environment which will permit sperm penetration to occur.

It has been known for many years that oocytes removed from mammalian ovarian follicles will resume meiosis spontaneously (without hormone additives) in a suitable tissue culture medium, but reports show that such oocytes have an extremely poor capacity for fertilisation and normal embryonic development. An analysis of published data by Cambridge workers (comprising more than 1,500 oocytes from mice, rabbits, sheep and cattle) showed that 40-60% of intra-follicular oocytes and less than 1% of extra-follicular oocytes developed into normal embryos when transferred to recipients. Extra-follicular maturation refers to those events associated with germinal vesicle breakdown (GVBD) and the resumption of meiosis. However, apart from such nuclear maturation, the process also includes changes within the oocyte's cytoplasm. Normal maturation as it occurs in-vivo includes all of the events which render the oocytes capable of fertilisation and competent to proceed with normal embryonic development. In cattle, events associated with nuclear maturation in oocytes have been reported by numerous authors, starting with the study of several mammalian species by Edwards (1965). The first work directed specifically at cattle was that of Sreenan, J. et al. (1968) J. Agric. Sci. Camb., 70, 183-185 who recorded that upon release from the follicles (3-6 mm diameter) 80% of oocytes reached metaphase II 24 h after incubation in tissue culture medium.

In the cow ovary, the oocyte destined for ovulation rapidly acquires developmental competence, the process involving far-reaching changes in many aspects of the oocyte's morphology and function. In effect, the egg cell is reprogrammed to enable it to support not only fertilisation but also the early stages of embryonic development. Although the term maturation was originally applied specifically to the morphological changes in the oocyte which included GVBD, condensation of the chromosomes and progression of meiosis through to the metaphase II stage and the extrusion of the first polar body, the term today covers equally important biochemical and physiological changes, including extensive changes in the pattern of protein synthesis, the transport of amino acids in the cell and carbohydrate metabolism. It is evident from the literature that gonadotrophins, steroids and cellular factors all interact to provide essential support for the oocyte during maturation in-vivo and there are several reports to suggest that the co-culture of bovine oocytes with follicle cells in-vitro may greatly enhance their capacity to undergo normal fertilisation (Fulka, J. and Motlik, J. (1980) Proc. 9th Int. Congr. Anim. Reprod. AI. (Madrid) 2, 55-62) and subsequent embryonic development. In sheep it has been demonstrated that there is a beneficial effect from adding additional cumulus cells to oocytes in culture (Staigmiller, R. B. and Moor, R. M. (1984) Gam. Res. 9, 221-229).

Current methods of IVF do not permit one to achieve a normal pregnancy rate in recipient cattle following transfer of the embryo resulting from IVF. Methods of providing a plentiful supply of cattle eggs and embryos at low cost would be of substantial value not only for research purposes but also for the commercial development of techniques such as cattle twinning by embryos transfer.

Workers have exposed cattle oocytes individually or in groups to sperm. In-vivo in the cow, fertilisation would normally occur in the presence of relatively few sperm in the oviduct. In cattle IVF, however, the commonly used sperm concentration is 0.5-5.0 x $10^6$/ml. Sperm-egg ratios used in some cattle IVF have been of the order of 10,000-20,000:1, although in some reports it has been reduced as low as 2,000:1 and for some bulls as low as 500:1. The maintenance of sperm viability at very low dilution rates is a matter of having an appropriate culture medium capable of supporting them. In-vivo in the cow, the oocyte encounters few sperm at the time of fertilisation and blocks to polyspermy have time to be expressed. With IVF, much larger sperm numbers surround the oocyte and with increasingly efficient capacitation procedures there is a possibility of several sperm penetrating the zona pellucida and vitelline membrane. The time that sperm and oocytes are co-cultured is a further consideration, since the blocks to polyspermy in oocytes matured in-vitro may not always be as effective as those matured in-vivo. Accordingly, it will be appreciated sperm:oocyte ratios and the time of sperm-oocyte culture should both be observed carefully in setting up an IVF system.

A problem observed in IVF in using large numbers of sperm is that the sperm tend to agglutinate in the sperm suspension used in IVF. Specifically, the sperm heads have a tendency to stick together and hence such agglutinated sperm are unavailable for potential fertilisation of the matured oocyte and hence the possibility of fertilisation is considerably reduced. The bull sperm head is about 8.5 lm long, 4.0 lm wide and 0.3-0.5 lm thick.

Although there are studies which have reported the successful culture of some early cleavage cattle embryos to the morula or blastocyst stage, the development block, which apparently occurs at the 8-16 cell stage normally precludes in-vitro development beyond that stage. Furthermore, only a minority of long-term embryos have shown regular cleavage and even that minority also invariably fail to develop further when transferred to recipient animals (Wright, R.W. and Bondioli, K.R. (1981) J. Anim. Sci., 53, 702-729). Cattle eggs cultured in-vitro at the 1 to 4 cell stage rarely progress beyond the 8-16 cell stage but those embryos cultured from the 8-16 cell stage can be expected to develop into morulae and blastocysts if they are developmentally competent. It appears that blocked development occurs at a time of prolonged cell cycle, DNA synthesis and a transition from maternal to zygotic control of development. Cells of the embryo apparently remain alive during the period of blocked development but they cannot be rescued once the block is initiated (Eyestone, W.H. and Furst, N.L. (1986) Theri., 25, 152).

Several authors have developed co-culture systems for use with single-cell and early stage cattle embryos. Such systems are developed on the principle that there is a positive effect on embryo development arising from cell-cell contact.

Some work has been done in evaluating the development of cattle embryos at the morula stage when co-cultured with uterine fibroblast cells (Wiemer et al., (1987) Theri., 27, 294) and J. Anim. Sci. 65 (Suppl.), 53 (Abs.)). Others have employed uterine endometrial cells and oviductal cells in co-culture studies.

The co-culture of early cattle embryos with oviductal cells has been reported (Eyestone et al. (1987) Theri., 28, 1-7): using 5-8 cell eggs from super-ovulated cattle, they reported 46% developing to the late morula or blastocyst stage of development. However, the subsequent developmental competency of these embryos was not examined.

It is an object of the present invention to provide a readily reproducible method of extracting oocytes from intact follicles and culturing the extra-follicular oocytes for IVF and which enables one to achieve normal pregnancy rates after transfer of embryos to recipient cattle resulting from said IVF.

It is a further object of the present invention to provide a method of separating sperm in a sperm suspension used in IVF wherein large numbers of sperm are used in the fertilisation step relative to the numbers of oocytes and which thereby increases the likelihood of fertilisation.

It is a still further object of the present invention to provide a method for the in-vitro culture of the early 15 bovine zygote (2-4 cell stage) which can be used as an alternative to bovine oviductal cells and which results in significant numbers of zygotes developing to the late morula or blastocyst stage of development.

According to the invention there is provided a method for the in vitro culture of the early bovine zygote to the late morula or blastocyst stage, which method comprises incubating intact Cumulus-Oocyte-Complex under non-static conditions in a medium containing greater than 10% by weight of heat-inactivated oestrus cow serum and greater than $4 \times 10^6$ additional folicle cells until the oocytes reach maturation, fertilising the oocytes with capacitated bull sperm and co-culturing the zygotes thereby obtained on a monolayer of cumulus cells for 6-9 days to enable them to reach the late morula or blastocyst stage of development.

Preferably, $5-7 \times 10^6$ additional follicle cells are added to the medium in which the oocyte can be matured and hereinafter referred to as the "maturation medium".

Further, preferably, the additional follicle cells are obtained from the medium in which the follicles were ruptured and rinsed. The follicle cells are preferably washed by centrifugation before addition to the maturation medium.

The maturation medium is preferably a synthetic culture medium such as Medium 199 (M 199) (Proc. Soc. ExP. Biol. Med., 73, 1 (1950)).

The maturation medium preferably contains 20% by weight of heat-inactivated oestrous cow serum.

The maturation medium used in the method according to the invention is free from added hormones.

Preferably, the follicles used for oocyte recovery are characterised by having a bright translucent appearance and good vascularisation.

Aiso preferably the ovaries, the source of the follicles, are maintained at a temperature which is not less than 30°C up to and including the time of extraction of the Cumulus-Oocyte-Complex.

Preferably, the pH of the maturation medium is in the range 7.3-7.6, but especially is maintained at a pH of 7.4.

Preferably, the oocytes are incubated for 24-26 h at a temperature in the range 38.5-39°C in an atmosphere of 5% $CO_2$ in air and under maximum humidity conditions.

The matured oocytes for insemination by capacitated bull sperm are preferably partially denuded of associated cumulus cells before contact with the sperm.

In general, four major criteria have to be met in order to achieve a fully efficient oocyte maturation system. These criteria are:

i) in the initial stages of culture, cell contact between the oocyte and the surrounding cumulus and follicle cells must be maintained;

ii) sufficient numbers of follicle cells (cumulus and follicle) are required to ensure an adequate flow of materials and energy into the maturing oocyte which brings about normal development of said oocyte;

iii) the follicle cells must be maintained in a healthy state during maturation by using a flux system which keeps them from sticking to the bottom of the maturation vessel; and

iv) the use of oestrous cow serum which ensures that the follicle cells produce all of the materials necessary for the oocyte during the culture period.

In order to ensure these criteria are met, care must be taken in follicle dissection to ensure that the Cumulus-Oocyte-Complex and its junctions remain undisturbed. The addition of follicle cells to the culture medium ensures that the requisite ingredients for maturation are secreted at a sufficient level. A non-static or flux culture system must be used in order that the follicle cells remain unattached to the culture vessel and thereby maintain their correct state of differentiation. It has been found that oestrous cow serum is a vital ingredient in the culture or maturation medium, possibly because of its steroid hormone and/or growth factor components. It is essential that the oestrous cow serum is taken from several heifers that are showing a natural heat and that it is carefully heat-inactivated before being sterilised and used in the maturation medium.

Preferably, after addition of the sperm suspension to the oocyte(s), the oocyte(s) are incubated in a $CO_2$ incubator at 39°C for 18-20 h, while maintaining a gas phase of 5% $CO_2$ in air, in an atmosphere of high relative humidity.

Preferably, a suspension of the capacitated bull sperm is passed through an orifice having a diameter in the range 15-30lm so as to separate the individual spermatozoa prior to its being added to the in-vitro matured oocytes.

The orifice suitably has a diameter in the range 22-27 lm, more especially 25 lm.

Further, preferably, the sperm suspension is pass through said orifice a number of times and especially up to five times.

The sperm suspension is suitably passed through a 25 gauge needle or a micropipette having a smallest diameter of 25 1m.

Preferably, the volume of sperm suspension passed through said orifice is 1 to 10 ll, more particularly 2 to 5 ll. Also preferably the concentration of sperm in the suspension is such that when added to a microdroplet containing the matured oocyte(s) in a suitable fertilisaton medium, one obtains a final concentration of sperm in the microdroplet of 1-1.5 x $10^6$/ml. Preferably, the ratio of sperm to oocyte used is 10,000-15,000:1. Preferably the microdroplet has a volume of 40-50 ll, more especially 46 ll.

The sperm prior to being subjected to the sperm separation step in accordance with the invention is preferably capacitated with heparin at 39°C in a $CO_2$ incubator.

Also preferably after addition of the sperm suspension to the oocyte(s), the oocyte(s) is/are incubated in a $CO_2$ incubator at 39°C for 18-20 hours, while maintaining a gas phase of 5% $CO_2$ in air, in an atmosphere of high relative humidity.

Preferably, the cumulus cells used in the culturing of the early bovine zygote are obtained from matured oocytes to be used for in-vitro fertilisation by partial denudation thereof. Such cumulus cells will be those which normally can be found surrounding healthy oocytes which show evidence of good cumulus cell expansion.

The cumulus cells for use in the method according to the invention are plated out in a culture vessel so as to establish a cumulus monolayer.

Further, preferably, the culture vessel is negatively charged so as to achieve a good establishment of said cumulus cell monolayer.

Preferably, co-culturing of the bovine zygote is carried out in accordance with the invention at 39°C in a gas phase of 5% $CO_2$ in air at maximum humidity.

Preferably, the medium in which the bovine zygote is co-cultured with the cumulus cells is replenished at intervals.

The bovine zygotes after 6-9 days of co-culture with the cumulus cells in accordance with the invention are evaluated and are either used for fresh transfers to a recipient cow or heifer or, alternatively, are stored frozen in liquid nitrogen for subsequent use.

By treating bovine extra-follicular oocytes prior to fertilisation and co-culturing the resulting early bovine zygote in accordance with the method of the invention enables one to achieve normal pregnancy rates in cattle using IVF and subsequent transfer of the embryo which has reached the blastocyst stage to a recipient cow or heifer.

Accordingly, the method according to the invention provides a reproducible means of ensuring fertilised cattle oocytes and a plentiful supply of embryos at low cost, with the obvious benefits to those concerned with cattle breeding.

The invention will be further illustrated by the following Example.

## EXAMPLE

### A) Collection of Ovaries and Oocytes

Ovaries were collected on a daily basis as required from heifers or cows slaughtered in a local abattoir and returned to the laboratory within one hour of slaughter. Appropriate records were made of the type of animals that contributed the ovaries.

The ovaries were stored temporarily in a Thermos (Trade Mark) flask in phosphate buffered saline (PBS) supplemented with 0.3% of bovine serum albumin (BSA) and 0.05 mg kanamycin/ml at 30°C. Every effort was made to ensure that the temperature did not fall below 30°C between the time the ovaries were collected and the time they arrived at the laboratory.

The ovaries were washed twice in fresh clean PBS and then dried lightly on filter paper before dissecting the intact follicles. The size of a follicle can vary from 2-6 mm in diameter. Follicles used for oocyte recovery should be characterised by having a bright translucent appearance and good vascularisation.

Follicles were isolated by dissection, and placed in Medium 199 (M 199) (Proc. Soc. Exp. Biol. Med., 73. 1 (1950)) which was held in small sterile Petri dishes held at 30°C in a sterile environment in a Laminair (Trade Mark) Flow Cabinet. The intact follicles were ruptured by way of a small scalpel blade and the Cumulus-Oocyte-Complete (COC) liberated from the follicles with the minimum of disturbance to the integrity of the complex. The ruptured follicles were washed in a dish of M 199 and the follicle remnants were discarded. The dish in which the ruptured follicles were rinsed served as the source of additional follicle cells that were added to oocytes during their maturation as hereinbelow described.

Each Cumulus-Oocyte-Complex was washed twice in fresh M 199 before being placed in an In-Vitro maturation medium. The room in which the oocyte recovery took place was kept as clean and sterile as possible. Also instruments used in follicle dissection and oocyte recovery were kept sterile.

### B) In-Vitro Maturation of the Primary Oocyte

The maturation medium used was M 199 which includes Earls salts, L-glutamine and 25 mM Hepes buffer (Cat. No. 041-2340-Gibco). The medium was supplemented with 20% oestrous cow serum (heat-inactivated) and trace amounts of antibiotics (100 i.u. penicillin and 100 lg streptomycin/ml). The pH was adjusted to 7.4 using either Hcl or NaOH.

Suitable oocytes were selected on the basis of the Cumulus-Oocyte-Complex and placed (20 in a group) in sterile Petri dishes each containing a 2 ml volume of the M 199 maturation medium.

To each 1 ml of maturation medium, was added $5\text{-}7 \times 10^6$ additional follicle cells, i.e., a total of $10\text{-}14 \times 10^6$ per 2 ml volume in the dish.

The source of these follicle cells was the medium in which the follicles were ruptured and rinsed. The cells were washed twice by centrifugation at 500 g for 5 minutes in M 199. The viability of these cells was checked, where necessary, using the Trypan Blue exclusion test (Sigma Chemicals Catalogue, 1987 issue). The number of cells added to medium can also be checked periodically using a haemocytometer slide so as to ensure that the required number of cells are always added.

The oocytes were matured in-vitro using a flux system for 24-26 h at 39°C on a gently rocking platform in an incubator in an atmosphere of 5% $CO_2$ in air at maximum humidity. The incubator in which the maturation was carried out was routinely cleaned and disinfected and the dishes and containers in which the maturation medium was located were also kept as sterile as possible.

The temperature of the incubator was monitored regularly and was not allowed to rise above 39°C - the optimum figure being within the range of 38.5-39°C.

The flow of $CO_2$ gas through the maturation system was carefully monitored so that it was at the appropriate rate at all times. The location of the incubator was not varied, since the operation of the incubator can be influenced by the ambient temperature of the room in which it is located.

After the 24-26 h period of maturation culture the oocytes were carefully checked for evidence of cumulus cell expansion. The pH of the maturation medium was carefully checked at the start and at the end of the culture period. Care was taken to ensure the temperature of the maturation medium was not subjected to any sudden reduction when removing the Petri dishes from the incubator.

## C) In-Vitro Capacitation of Bull Sperm

Straws of frozen bull semen (egg yolk-lactose-glycerol) were obtained locally (Dublin District Milk Board, Enfield, County Meath, Ireland) and were thawed out by immersing the straws in a water-bath held at $37^\circ$ C for 30-40 seconds. A check of the sperm under the Biological Microscope was made to ensure that it was showing satisfactory motility. A mixture of sperm from two bulls was used. It is essential to note details of the bull used and the type of diluent used in the straw since this may affect capacitation and the subsequent result.

A swim-up separation technique (Parrish, J.J. et al. (1985) Ther., 23, 216) was employed in order to obtain the more motile sperm. To achieve this volumes of 0.2-0.25 ml of thawed semen were layered under 1 ml of modified (calcium ion free) Tyrode's medium (pH 7.4) which was used as the capacitation medium.

The layering of semen was done in an open test-tube which was placed in the $CO_2$ Incubator held at $39^\circ$ C and incubated in 5% $CO_2$ in air for a period of 60 minutes.

After this period of incubation, 0.7-0.8 ml of medium was removed from the top of each tube and the sperm suspensions from all tubes were combined. The pooled sperm suspension was then centrifuged at 500 g for 10 minutes, after which the supernatant was drawn off to leave a sperm pellet. The sperm pellet was resuspended in 4-5 ml of capacitation medium and again centrifuged for 7-10 minutes before the supernatant was again drawn off.

Finally sufficient capacitation medium was added to the sperm pellet to give an estimated sperm density of $100 \times 10^6$ sperm/ml. This density figure can be checked using a haemocytometer. A volume of 50 ll of capacitation medium containing 200 lg heparin/ml was then added to 50 ll of sperm suspension to give a final sperm concentration of $50 \times 10^6$/ml.

The sperm suspension was then incubated for 15 minutes at $39^\circ$ C in the $CO_2$ incubator before being diluted 1:1 with capacitation medium to give a final dilution figure of $25 \times 10^6$ sperm cells/ml. Care was taken to ensure that the capacitation medium had a pH of 7.4.

## D) In-Vitro Fertilisation (IVF) Procedures

Fertilisation medium (Tyrode's (Bavister, B.D. (1981). IN: Fertilization and Embryonic Development In-Vitro (Eds. Mastroianni, L. and Biggers, J.D.) Plenon Pres., London, 41-60) in microdroplets, 46 ll in volume, was prepared in sterile Petri dishes with the medium held at a pH of 7.8.

Optionally, immediately prior to preparing the fertilisation microdroplets, 10 mM hypotaurine and 20 mM penicillamine were added to the medium.

The microdroplets were covered by a sterile light mineral oil (Sigma M3516) and the dish containing the droplets placed in the $CO_2$ incubator for one hour to enable the fertilisation medium to be equilibrated by exposure to the 5% $CO_2$ atmosphere.

Oocytes for insemination were selected on the basis of their morphological appearance and washed once in Wash 2 Medium (Modified Tyrode's) before being "cleaned" until only a few layers of cumulus cells remained surrounding them. This cleaning was done by mechanically stripping away cumulus cells - by drawing the oocytes in and out of appropriately sized sterile glass pipettes. The cumulus cells thus stripped away were used in the co-culture of the early bovine zygote on a monolayer of culmulus cells in accordance with the invention and as hereinbelow described in Section E). The oocytes were then washed twice in fresh Wash 2 Medium.

A group of five oocytes was then arranged in each of the fertilisation droplets before a volume of 2 to 5 ll of sperm suspension was added so as to achieve a final concentration of sperm in the droplet of $1-1.5 \times 10^6$/ml. This works out at a ratio of one oocyte per 10,000 to 15,000 sperm. The spermatozoa in the sperm suspension were separated by passing the suspension through a 25 gauge needle (x3).

The Petri dish containing the droplets was then placed in the $CO_2$ incubator at $39^\circ$ C for 18-20 h, maintaining a gas phase of 5% $CO_2$ in air and an appropriate high relative humidity.

In carrying out the IVF, strict aseptic precautions were observed throughout to avoid any contamination of the medium, by performing the IVF in a sterile cabinet at $30^\circ$ C in a sterile atmosphere.

## E) In-Vitro Co-Culture of the Early Bovine Embryo (2-4 cell stage) on a Cumulus Monolayer

## Preparation of Cumulus Monolayer

A maturation medium consisting of M 199 supplemented with 10% (v/v) of oestrous cow serum and antibiotics was allowed to equilibrate overnight. The antibiotics used were 100 i.u. penicillin and streptomycin 100 lg/ml. On the following day, microdroplets 50 ll in volume were prepared in a sterile culture dish and covered with 10 ml of sterile mineral oil (Sigma Chemicals). The medium was then allowed to stabilise for a period of two hours.

The cumulus cells were obtained from the "wash-dishes" which were used at the time of preparing the matured oocytes for insemination by the partial denudation of the cumulus cells as described in section D).

The suspension of cumulus cells thus obtained was centrifuged at 500 x g and the pellet resuspended in M 199 supplemented with 10% oestrous cow serum and antibiotics as for the preparation of the microdroplets above. The centrifugation procedure was then repeated.

Pellets from more than one tube were mixed using an 18 G needle and a 1 ml sterile syringe and the cells were resuspended in 0.5 ml of medium in a Vacutainer (Trade Mark) tube. The 15 ll microdroplets were then innoculated with 6 ll of suspended cells and allowed to remain in a $CO_2$ incubator at 39°C for 36 hours to enable plating to occur on the bottom of the dish. The culture dishes were negatively charged so as to achieve a good establishment of the cumulus monolayer.


Co-culture procedure

Early bovine embryos (zygotes) (2-4 cells) showing regular blastomeres were placed in the microdroplets of medium in groups of 5-10 and cultured at 39°C in a gas phase of 5% $CO_2$ in air at maximum humidity.

The medium was replenished at intervals of 24 hours by removing 30 ll volumes of medium from the microdroplets and replacing it with 30 ll of fresh medium. The replacement medium was equivalent to the medium used for the previous steps described in this section except that the antibiotic cover was provided by gentamycin sulphate (50 lg/ml) and not penicillin/streptomycin.

The fertilised bovine eggs (zygotes) were allowed to remain on the cumulus monolayer for 6-9 days to permit them to reach the blastocyst stage of development, at which time they were evaluated and either used for fresh transfers or stored frozen in liquid nitrogen for subsequent use as hereinbelow described.


F) Evaluation, Storage and Transfer of IVF Embryo to Recipients

Embryos produced by the In-Vitro method of Section E) above were evaluated according to normal procedures established in the laboratory and as described by Kennedy, L.G., et al. Ther. 19, 823-832 using morphological examination. Embryos so assessed as being top grade were suitable for freezing in straws for storage in liquid nitrogen. A one-step freezing procedure was employed so that the embryos could be thawed and transferred under farm conditions.

For the one-step freezing, the medium used was PBS supplemented with oestrous cow serum (heat inactivated) at a level of 15% (v/v), antibiotics (100 i.u. penicillin and 100 lg streptomycin/ml) and a cryoprotectant (glycerol) at a concentration of 1.4 M. The PBS may also be supplemented with fetal calf serum. A sucrose solution was used for diluting out the cryoprotectant after thawing in a concentration of 0.5 M in PBS supplemented with 15% serum.

Each embryo was placed directly in the freezing medium (PBS and 15% serum and 1.4 M glycerol) at normal room temperature (15-25°C). After 15 min. equilibration, each embryo was loaded into the mid-portion of a 0.5 ml plastics straw (IMV of France) in a small volume (0.05 ml) of the freezing medium separated by air bubbles on each side from larger volumes of 0.5 M sucrose diluting medium. When the straw was filled, the open end was heat-sealed and the plug-end inscribed with the identity of the embryo (bull and dam information). In the freezing operations, the straw was exposed directly to a temperature of -7°C and held at that temperature for one minute prior to seeding. After the seeding, the temperature was reduced at the rate of 0.3°C/min. until a temperature of -30°C was reached. At that temperature, the straw was plunged directly into liquid nitrogen (-196°C) and stored at that temperature until required.

For thawing, the straw was plunged directly into water held at 37°C for one minute. The fluid contents of the straw were then mixed by shaking rapidly three or four times (using a motion as in shaking down a clinical thermometer). Transfer of the embryo was carried out 10 min. after thawing by normal non-surgical procedures as developed by Gordon, I. (1983) Controlled Breeding in Farm Animals, Pergamon Press, Oxford.

Transfer of embryos (fresh or frozen) varies according to whether the objective is to establish a single or a twin-pregnancy. For single pregnancy initiation, the recipient animal must be at exact synchrony with the embryo stage. The ovary containing the corpus luteum is identified and the embryo is transferred to the uterine horn associated with that ovary. The 0.5 ml straw is loaded into a standard Cassou (Trade Mark) Inseminating instrument (IMV of France) and introduced through the cervix. For initiation of a single pregnancy the embryo was deposited in the mid-horn position or beyond. For the initiation of a twin-pregnancy, two embryos were employed, one being deposited in each horn. Only cows were used as recipients for twins. In the transfer procedure, care was taken at all points to maintain sterility in the handling and manipulation of the straw.

G) Pregnancy Monitoring

Monitoring of pregnancy for information on embryo/foetal survival was carried out during early pregnancy using ultra-sound scanning. In the absence of information about "repeating", this can be done from 30 days of gestation onwards. "Repeating" alludes to an animal coming into "heat", thus indicating it is not pregnant. After mid-pregnancy, an oestrogen assay as developed by MacDonnell, H. et al. (1984) Proc. 10th Int. Congr. Anim. Reprod. A.I., 2, 76 for twin-pregnancy diagnosis was employed to determine whether twins were present.

It was found that normal pregnancy rates could be established in cows and heifers following the IVF and subsequent embryo transfer procedures outlined above. This evidence of a normal pregnancy rate in the recipient cattle is an indication that a reliable in-vitro fertilisation system has been established in accordance with the invention which may be a means of supplying cattle embryos cheaply, with the obvious benefits to farmers and, indeed, cattle breeding in general.

**Claims**

1. A method for the in-vitro culture of the early bovine zygote to the late morula or blastocyst stage, which method comprises incubating intact Cumulus-Oocyte-Complex under non-static conditions in a medium containing greater than 10% by weight of heat-inactivated oestrous cow serum and greater than $4 \times 10^6$ additional follicle cells until the oocytes reach maturation, fertilising the oocytes with capacitated bull sperm and co-culturing the zygotes thereby obtained on a monolayer of cumulus cells for 6-9 days to enable them to reach the late morula or blastocyst stage of development.

2. A method according to Claim 1, wherein 5-7 $\times 10^6$ additional follicle cells are added to the medium in which the oocytes are matured.

3. A method according to Claim 1 or 2, wherein the medium in which the oocytes are matured contains 20% by weight of heat-inactivated oestrous cow serum.

4. A method according to any preceding claim, wherein the pH of the maturation medium is in the range of 7.3-7.6.

5. A method according to Claim 4, wherein the pH of the maturation medium is 7.4.

6. A method according to any preceding claim, wherein the oocytes are incubated for 24-26 hours, at a temperature in the range 38.5-39°C in an atmosphere of 5% $CO_2$ in air, at maximum humidity.

7. A method according to any preceding claim, wherein a suspension of the capacitated bull sperm is passed through an orifice having a diameter in the range 15-30 lm so as to separate the individual spermatozoa prior to its being added to the in-vitro matured oocytes.

8. A method according to Claim 7, wherein the orifice has a diameter in the range 22-27 lm.

9. A method according to Claim 7 or 8, wherein the sperm suspension is passed through the orifice a number of times.

10. A method according to any preceding claim, wherein the cumulus cells used in the culturing of the zygote are obtained from matured oocytes used for the fertilisation step, by partial denudation thereof.

11. A method according to any preceding claim, wherein the cumulus cells are plated out in a culture vessel so as to establish the cumulus cell monolayer.

12. A method according to any preceding claims, wherein the culturing of the zygote is carried out at 39°C in a gas phase of 5% $CO_2$ in air, at maximum humidity.

13. A method according to any preceding claim, wherein the medium in which the zygote is co-cultured with the cumulus cells is replenished at intervals.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | BIOLOGY OF REPRODUCTION, vol. 37, 20th August 1987, pages 859-866; R. PRATHER et al.: "Nuclear transplantation in the bovine embryo: assessment of donor nuclei and recipient oocyte" * Page 860, paragraph 2 * | 1-13 | C 12 N 5/00 |
| A | THERIOGENOLOGY, vol. 27, no. 1, January 1987, page 228; W.H. EYESTONE et al.: "Co-culture of early bovine embryos with oviductal epithelium" * Whole article * | 1-13 | |
| A | J. REPRODUCTION AND FERTICITY, vol. 81, no. 1, 1987, pages 23-28; F. GANDOLFI et al.: "Stimulation of early embryonic development in the sheep by co-culture with oviduct epithelial cells" * Whole document * | 1-13 | |
| A | J. REPRODUCTION AND FERTILITY, vol. 72, no. 2, 1984, pages 479-485, Journals of Reproduction & Fertility Ltd, GB; S. CAMOUS et al.: "Cleavage beyond the block stage and survival after transfer of early bovine embryos cultured with trophoblastic vesicles" * Whole article * | 1-13 | TECHNICAL FIELDS SEARCHED (Int. Cl.4) C 12 N 5 C 12 N 15 |

The present search report has been drawn up for all claims

| Place of search | Date of completion of the search | Examiner |
|---|---|---|
| THE HAGUE | 07-08-1989 | ROSDY M.K.P. |

EPO FORM 1503 03.82 (P0401)